(19) Europäisches Patentamt
European Patent Office
Office européen des brevets

(11) **EP 2 524 706 B1**

(12) **EUROPÄISCHE PATENTSCHRIFT**

(45) Veröffentlichungstag und Bekanntmachung des Hinweises auf die Patenterteilung:
**23.07.2014   Patentblatt 2014/30**

(21) Anmeldenummer: **11004167.0**

(22) Anmeldetag: **19.05.2011**

(51) Int Cl.:
*A61L 15/24* *(2006.01)*          *A61L 15/60* *(2006.01)*
*A61F 13/00* *(2006.01)*

(54) **Sterile Wundauflage mit einem synthethischen Dreiblock-Elastomer**

Sterilized wound dressing comprising a triblock-elastomeric component

Pansement stérile comprenant un composé élastomère tribloc

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.11.2012   Patentblatt 2012/47**

(73) Patentinhaber: **Lohmann & Rauscher GmbH & Co. KG**
**56567 Neuwied (DE)**

(72) Erfinder: **Gorka, Marius-Thomas**
**56218 Mülheim-Kärlich (DE)**

(74) Vertreter: **Leinweber & Zimmermann**
**Rosental 7, II. Aufgang**
**80331 München (DE)**

(56) Entgegenhaltungen:
**FR-A1- 2 783 412**

**Beschreibung**

Die Erfindung betrifft eine sterile Wundauflage mit einem Träger und einer elastomeren Polymermatrix als Wundkontaktschicht.

Fetthaltige Wundauflagen werden seit Jahren erfolgreich für die lokale Behandlung von traumatischen Wunden, wie etwa Schnitt- und Schürfwunden, und chronischen Wunden, wie etwa Dekubitus, Ulcus cruris u. dgl., sowie Verbrennungen bei Menschen und Tieren, eingesetzt.

Ein mit Fett imprägniertes Tüll Lomatuell H, der Fa. L&R, stellt eines der häufig zur medizinischen Versorgung der Hautwunden verwendeten Produkte dar. Dieses Produkt, das aus einem großmaschigen mit einem Fettkörper auf Vaselinebasis imprägnierten Baumwollgewebe gebildet ist, weist dennoch Nachteile auf, wie beispielsweise Verlust von Fettkörpern an die Handhabungswerkzeuge (wie etwa Handschuhe) und an die Wunde und Wundumgebung. Bei Kontakt mit der Wunde wird die Vaseline aufgrund der Temperaturerhöhung weich und neigt dazu, an die Peripherie des Verbands gedrängt zu werden.

Dieses Phänomen führt häufig zum Freilegen des Trägers und zum direkten Kontakt mit der Wunde. Das Verkleben des Trägers mit der Wunde führt oft zum traumatischen Verbandwechsel und zur Störung des Wundheilungsprozesses.

Andere im Handel befindliche Produkte, wie z.B. der Verband der Fa. Johnson und Johnson ADAPTIC, der ein mit einer öl-in-Wasser-Emulsion getränkter Viskosestrickstoff ist, oder das Produkt JELONET (Fa. Smith und Nephew), das eine mit Paraffin getränkte Baumwollgaze ist, erfüllen die gleiche Zweckbestimmung wie das Lomatuell H, weisen jedoch ebenfalls ein nachteiliges Verhalten in Bezug auf den Verlust von Fettkörpern auf.

Ferner scheint es, daß die bekannten Verbände vom gefetteten Tüll-Typ im Gebrauch zu hydrophob sind und ein unerwünschtes Austrocknen der Wunde begünstigen.

Es entspricht dem allgemeinen Wissensstand, daß die Zugabe einer kleinen Menge von hydrophilen Substanzen aus der Hydrokolloidgruppe wie etwa von CMC in Dispersion zu einer hydrophoben elastomeren Matrix, die Hydrophilie der Matrix nach Kontakt mit dem Wundexsudat durch das Bilden eines Gels auf der Oberfläche partiell erhöht. Diese Mischung aus hydrophoben und hydrophilen Eigenschaften der Oberfläche der Matrix, die mit der Wunde in direkten Kontakt kommt, führt zu einem für den Heilungsprozeß der Wunde äußerst günstigen Ergebnis: ein optimaler oberflächlich aufrechterhaltener Feuchtigkeitsgrad und die Gegenwart von Fettkörpern, die die Struktur des Verbands isolieren, führen zu schnellerer Wundheilung und vollkommener Nichthaftung der Kompresse an der Wunde.

In der EP 1 691 851 B1 ist ein Wundverband beschrieben, der ein mit Öffnungen versehenes flüssigkeitsdurchlässiges Substrat und eine absorbierende (mindestens 50 % der Trockenmasse) nichthaftende Polymerzusammensetzung aufweist, die eine hydrophobe organische Polymermatrix, einen Weichmacher und hydrophile organische Mikroteilchen sowie ggf. bioaktive Mittel enthält. In der genannten Schrift werden synthetische und natürliche bioaktive Mittel beschrieben. Beim Einsatz dieser Mittel hat es sich als problematisch erwiesen, daß das Absorptionsmittel (Superabsorber) im Verband die Flüssigkeit in Form eines Gels bindet. Durch den Quellungsvorgang werden die Öffnungen für Exsudat (z. B. in dickflüssiger Form) nahezu unpassierbar. Ein freier Abtransport des Exsudats in den saugenden Sekundärverband kann dann behindert werden. Dies kann unter Umständen eine Mazeration der Wunde hervorrufen.

Das Verkleben des Verbands mit der Wunde kann durch Einsatz einer sterilen, nichtklebenden Kompresse gemäß der EP 1 143 895 B1 verhindert werden. Diese Kompresse enthält eine polymere Zusammensetzung aus einem thermoplastischen Elastomer, einem öligen Weichmacher, Vaseline und hydrophilen Teilchen eines Hydrokolloids (CMC, Alginat, Pektin) und ggf. einen Wirkstoff. Gemäß einer bevorzugten Ausführungsform der in dieser Schrift beschriebenen Erfindung umfaßt die elastomere Matrix ein Dreiblock-Elastomer, wobei die Konzentration der Elastomere mindestens zwischen 3,2 % und 8,8 % betragen soll, damit die Verwendung einer kohäsiven, elastischen, in feuchtem Medium stabilen polymeren Matrix gewährleistet werden kann.

Es ist wünschenswert nur einen sehr eingeschränkten direkten Kontakt zwischen elastomerem Polymer und Wunde zuzulassen, wobei der Kontakt im Wesentlichen über die öligen Verbindungen erfolgt, die vom lebenden Gewebe der Wunde besser vertragen werden als die eingesetzten Elastomere. Anderseits ist der Einsatz der Polymere unbedingt notwendig, wobei der Gesamtpolymeranteil gemäß EP 1 143 895 mind. 3,2 % beträgt, um eine ausreichende Kohäsion und Elastizität der gesamten Matrix zu gewährleisten.

In der EP 127 229 B1 ist eine antiseptische Kompresse beschrieben, die eine Wundkontaktschicht aus einer elastomeren Matrix, einem unpolaren Öl, einem Hydrokolloid (Natriumcarboxymethylcellulose) als Dispersion und mind. einem Tensid (Tween 80), ggf. mit mind. einem antimikrobiellen Mittel (Silbersulfadiazin), aufweist. Bei einer bevorzugten Ausführungsform der in der genannten Schrift beschriebenen Erfindung weist eine Kompresse eine hohe Gesamtkonzentration des synthetischen Dreiblock-Elastomers von über 4,6 Gewichtsteilen auf. Das ist im Hinblick auf die angestrebte Vermeidung des direkten Kontakts zwischen Elastomer und Wunde problematisch. Ferner ist auch die Verwendung von Tensiden nicht unproblematisch, da sie ein gewisses zytotoxisches Potential besitzen.

EP 0 521 761 beschreibt einen nichtklebenden hydrophoben Okklusionsverband, der aus einem mit der Matrixoberfläche verbundenen Träger besteht, wobei die elastomere Matrix mindestens 10-30 Gewichtsteile eines Blockcopolymers und 70-90 Gewichtsteile eines Weichmachers, bevorzugt Vaseline, enthält.

Wundauflagen nach dem Oberbegriff des Patentanspruchs 1 sind in der FR 2 783 412 A1 angesehen.

Damit alle diese Wundauflagen zur Förderung der Heilung ohne Risiko von mikrobieller Verschmutzung verwendet werden können, müssen sie zuvor sterilisiert werden. Wundinfektionen verzögern nachweislich die Heilung. Sie werden durch pathogene Keime verursacht, die in die Wunde (ggf. über die Wundauflage) eindringen, sich dort vermehren und dabei Giftstoffe erzeugen, die sowohl auf das Wundgewebe als auch auf den gesamten Organismus wirken.

Es bestehen unterschiedliche Techniken, um verunreinigende Mikroorganismen zu zerstören. Neben der Sterilisation durch gesättigten Dampf oder durch trockene Wärme, wird routinemäßig die Sterilisation durch Gas (Ethylenoxid, Formaldehyd) oder die Sterilisation durch Bestrahlung angewendet. Jedoch ist keine dieser Techniken für die Herstellung von Produkten, insbesondere jenen mit pharmazeutischen Anwendungen und insbesondere Produkten, welche eine fetthaltige elastomere Matrix enthalten, vorbehaltlos geeignet. So ist die Sterilisation durch gesättigten Dampf oder Trockenwärme nicht anwendbar, da die elastomere Matrix und das Hydrokolloid die hohen Temperaturen und die extrem erhöhte Luftfeuchtigkeit nur schlecht vertragen.

Ebenfalls wird die Sterilisation durch Gas im Allgemeinen aufgrund des innewohnenden Risikos der Anwesenheit von Restgas in derartigen Wundauflagen vermieden. Außerdem ist es bei dieser Technik im Allgemeinen nicht möglich, eine Verteilung des Sterilisierungsmittels über das gesamte Volumen der elastomeren Polymermasse zu erhalten, was ihre Wirksamkeit eingrenzt.

Im Allgemeinen verhindert auch der Einsatz einer Sterilbarriere (primärer Packstoff), der die Sterilität des Produkts bei der Vermarktung bis zu der Anwendung am Patienten bewahrt, die Anwendung der meisten o. g. Sterilisationsverfahren, da der Packstoff als gasdicht ausgewählt wurde, um den oxidierenden Einfluß des Luftsauerstoffs sowie den Einfluß der Luftfeuchtigkeit auf das in der Matrix dispergierte hygroskopische Hydrokolloid zu unterbinden.

Die im Allgemeinen für die Sterilisation von derartigen Wundverbänden verwendete Technik ist folglich die Sterilisation durch Bestrahlung, welche somit eine sehr wirksame Sterilisation bis ins Innere des Produkts sicherstellt. Zwei Arten von Strahlungen können zu diesem Zweck verwendet werden, nämlich β- und γ-Strahlung.

Die Sterilisierungsdosis wird als Funktion der anfänglichen mikrobiologischen Belastung (*Bioburden*), das heißt, der Menge an Keimen, die vor der Sterilisation auf/im Produkt vorhanden ist, im Rahmen einer Dosisbestimmung angepaßt.

Um eine wirksame Dekontamination mit einer ausreichenden Sicherheitsspanne sicherzustellen, wird auf die zu sterilisierenden Produkte im Allgemeinen eine mittlere Dosis von 25 kGray angewendet. In der Praxis erhält ein Produkt eine Menge, die zwischen 25 und 40 kGray entsprechend dem verwendeten Verfahren variiert.

Diese beiden bekannten Techniken der Strahlensterilisation haben jedoch auch ungewünschte Wirkungen auf die behandelte elastomere Matrix. Insbesondere ist die Energie, die durch diese Strahlen in die Matrix eingebracht wird, ausreichend hoch, um die Verbindungen Kohlenstoff-Kohlenstoff und Kohlenstoff-Wasserstoff der verwendeten Elastomere zu zerbrechen und ebenfalls Unterbrechungen der Ketten in diesen polymeren Makromolekülen und Verminderungen ihrer mittleren molekularen Masse hervorzurufen, die ihre Eigenschaften, insbesondere ihre Kohäsionsfähigkeit, beeinflussen/herabsetzen.

Folglich sind diese o. g. Produkte, entweder auf Grund von Schwierigkeiten in Verbindung mit der Handhabung beim Anlegen oder Abnehmen des Verbands oder aufgrund eines relativ hohen Polymer-Preises, oder einer gewissen Gewebeunverträglichkeit der elastomeren Polymere, oder auch durch den Verlust der Kohäsion nach der Strahlensterilisation, nicht vollkommen zufriedenstellend.

Angesichts der vorstehend beschriebenen Probleme im Stand der Technik liegt der Erfindung die Aufgabe zugrunde, eine besonders bioverträgliche, nichtklebende (ggf. leicht haftende) Wundauflage auf Basis einer fett- und hydrokolloidhaltigen elastomeren Matrix mit verbesserter Beständigkeit gegenüber Strahlensterilisation für die Behandlung von traumatischen und chronischen Wunden sowie Verbrennungen zur Verfügung zu stellen.

Erfindungsgemäß wird diese Aufgabe durch die im kennzeichnenden Teil des Patentanspruchs 1 angegebene Weiterbildung der bekannten Wundauflagen gelöst.

Erfindungsgemäß werden thermoplastische Elastomere (TPE) bzw. Elastomermischungen mit besonders hohem Molekulargewicht eingesetzt, um den negativen Auswirkungen der Strahlensterilisation auf die Kohäsion der Matrix entgegenzuwirken. Überraschenderweise wurde dabei festgestellt, daß der Zusatz/Einsatz von den bevorzugten ultrahochmolekularen Elastomeren, wie Septon 4077, die Kohäsion nach der Sterilisation erheblich verbessert.

Bedingt durch den Inhalt an relativ temperaturempfindlichen Komponenten wie Vaseline oder CMC, in der Matrix, ist die Höhe der Verarbeitungstemperatur limitiert (bevorzugt max. ca. 140-145°C). Die mit dem Einsatz eines ultrahochmolekularen Elastomers erwartete Steigerung der Verarbeitungstemperatur ist aber nur geringfügig und liegt im akzeptablen Bereich.

Die Wundkontaktschicht einer erfindungsgemäßen Wundauflage umfaßt vorzugsweise neben einer organischen polymeren Matrix einen Weichmacher (*plasticizer*) und hydrophile organische bzw. anorganische Mikropartikel, die bei Kontakt mit wäßriger Lösung ein Gel bilden.

Nach einem der erfindungsgemäßen Merkmale umfaßt die Wundauflage ein flexibles offenmaschiges Gewebe, wobei das Gewebe Fasern einschließt, die so mit einem elastomeren, kohäsiven und nichtklebenden Gel/Matrix umhüllt sind, daß die Maschen im wesentlichen nicht verstopft belassen werden bzw. durchlässig bleiben.

Die polymere Matrix kann ein synthetisches thermoplastisches hydrophobes Dreiblockcopolymer A-B-A enthalten. Dabei enthält die polymere Matrix vorzugsweise nicht mehr als 3,2 Gewichtsteile, insbes. nicht mehr als 2,6 Gewichtsteile eines Blockpolymers, wobei der terminale Block A vom Polystyroltyp und der zentrale Block B vom gesättigten Polyolefintyp sein kann und der Styrenanteil zwischen 25 und 40 % beträgt.

Um eine erfindungsgemäße hydrophobe Matrix mit ausreichender Kohäsion auch nach der Strahlensterilisation zu erhalten, werden Dreiblock-Elastomere vom Typ SEBS oder SEPS und/oder SEEPS mit hohem und ultrahohem Molekulargewicht eingesetzt.

Zweckmäßig werden hydrierte Copolymere Polystyren-Polyethylen-Polybutylen-Polystyrene (SEBS, z. B. G1651, MD 9633, Kraton) eingesetzt. Im Rahmen der Erfindung werden hydrierte Copolymere Polystyren-b-Poly(ethylen/propylen)-b-Polystyrene (SEEPS, Septon 4055 und Septon 4077, Kuraray) bevorzugt.

Um eine erfindungsgemäße hydrophobe Matrix zu erhalten, wird mindestens ein Dreiblock-Elastomer vom Typ SEBS oder SEPS, besonders SEEPS mit sehr hohem Molekulargewicht (mindestens 200.000 Dalton, bevorzugt mindestens 400.000 Dalton) und einer Brookfield-Viskosität von mindestens 5000 mPas (für eine 10%ige Lösung in Toluol bei 30°C) gewählt.

Derartige polymere Zusammensetzungen gestatten den guten Einschluß der Fasern des Gewebes als Träger, die von der Wunde solange komplett isoliert bleiben; somit wird zu keiner Zeit die Herstellung eines direkten Kontakts zwischen Faser und regenerierten Geweben riskiert, der zu einem Faser-Einschluß in die Wunde mit der Folge einer schmerzhaften Zerstörung der Gewebe beim Abziehen des Verbands führen könnte.

Durch Einsatz von einer Mischung der hochmolekularen und ultrahochmolekularen thermoplastischen Elastomere (TPE) erhält man einerseits eine sehr weiche Kompresse die sich gut der zu schützenden Oberfläche anpaßt und die andererseits aufgrund der starken Kohäsion und der Elastizität der Matrix auch nach der Strahlensterilisation bei erfindungsgemäßer Anwendung auf der Wunde zu keiner Zeit so schadhaft wird, daß die Fasern offengelegt werden und in direkten Kontakt mit der Wunde treten. Dabei beträgt der Anteil des ultrahochmolekularen Polymers mit einem Molekulargewicht von mehr als 300.000 Dalton am Gesamtpolymeranteil zum Erhalt einer noch ausreichenden Verarbeitbarkeit weniger als 50 Gew.-%, insbesondere weniger als 25 Gew.%. Die angestrebte Verbesserung der Sterilisationsbeständigkeit bei Strahlensterilisation wird erreicht, wenn der Anteil des ultrahochmolekularen Polymers mit einem Molekulargewicht von mehr als 300.000 Dalton, insbesondere etwa 400.000 bis 450.000 Dalton, am Gesamtpolymeranteil mehr als 5 %, insbesondere mehr als 10 %, besonders bevorzugt 20 % oder mehr, beträgt.

Durch den hohen Anteil an öligem Weichmacher, der vorzugsweise ausgehend von einem Gemisch von Mineralöl und Vaseline erhalten wird, in Kombination mit einem sehr niedrigen Anteil an Polymeren/Elastomeren verhält sich die Matrix außerdem wie ein purer Fettkörper, wodurch eine sehr gute Gewebeverträglichkeit und Eigenschaften der Nichthaftung an der Oberfläche der Kompresse gewährleistet werden.

Unter den Produkten/Weichmachern, die zur Plastifizierung des Elastomers gut geeignet sind, kann insbes. auf bei Raumtemperatur flüssige oder feste Fettkörper, insbes. Paraffinöle, medizinische Weißöle, Mineralöle, Salbenparaffine, Vaseline, Silikonöle oder Silikonfette bzw. Wachse, hingewiesen werden, sowie auf Mischungen davon. Bevorzugt werden Weichmacher, wie Vaseline, deren Tropfpunkt zwischen 35 und 70 °C liegt. Bevorzugt werden auch medizinische Weißöle, deren Reinheitsanforderungen der Ph. Eur. entsprechen.

Die Matrix kann auch Antioxidantien enthalten. Als geeignete Antioxidantien können die Schwefel-Antioxidantien genannt werden, wie zum Beispiel das von der Firma Akzo Nobel chemicals unter der Verkaufsbezeichnung PERKACIT ZDBC vermarktete Zink-Dibutyldithiocarbamat, und/oder die Phenol-Antioxidantien, wie zum Beispiel die unter den Verkaufsbezeichnungen IRGANOX®1010, IRGANOX®565, IRGANOX®1035 von der Firma BASF vermarkteten Produkte.

Im Rahmen der vorliegenden Erfindung wird die Verbindung aus IRGANOX®1010 bevorzugt.

Die Wundkontaktschicht einer erfindungsgemäßen Wundauflage kann ferner ein Additiv, das aus der Gruppe bestehend aus einem weiteren Stabilisator, Extrudierhilfe, Füllstoff, Pigment, Farbstoff, Vernetzungsmittel, geruchshemmendem Mittel, Klebrigmacher, Verträglichkeitsvermittler und Kombination davon ausgewählt ist, umfassen.

Als hydrophile organische bzw. anorganische Mikropartikel, die Wasser binden und dabei gelieren, können z. B. die allgemein bekannten Hydrokolloide (CMC, Alginate, Gelatine, Xanthane, Pektine), aber auch Silikate, wie Bentonite, Aerosile oder Superabsorber, eingesetzt werden. Bevorzugt werden Mikropartikel mit einem Durchmesser von 50 bis 300 $\mu$m (unter Annahme einer sphärischen Form), insbes. mit einem Durchmesser von 50 bis 200 $\mu$m.

Das in der elastomeren Matrix in einer relativ geringen Menge dispergierte Hydrokolloid gestattet den Erhalt eines leicht hydrophilen Charakters, der für die Aufrechterhaltung einer wundheilungsfördernden feuchten Wundumgebung ausreichend ist, der allerdings nicht ausreicht, um das Gel in die Lage zu versetzen, viel Wasser zu absorbieren. In der Tat ist dieses Absorptionsvermögen nicht wünschenswert, da es zu einem Quellen des Gels führt, das eine schrittweise Verstopfung der in der Struktur der Kompresse belassenen Öffnungen hervorrufen würde. Die Kompresse würde demnach okklusiv werden, was somit die Möglichkeit der Beseitigung der Exsudate unter Herbeiführung eines Mazerationsrisikos unterdrücken würde, in der Kontaktschicht ausgearbeitete Passagen verstopft und eine Mazeration der Wunde hervorrufen kann.

Gemäß der vorliegenden Erfindung wird eine polymere Zusammensetzung bereitgestellt, die eine nicht absorbierende elastomere Matrix/Wundkontaktschicht umfaßt.

In Bezug auf die elastomere Polymermatrix bedeutet der Ausdruck "nicht absorbierend", daß die hydrophobe Matrix weniger als 35 %, vorzugsweise weniger als 25 % einer Salin-Lösung (wäßrige 0,8 % NaCl-Lösung), nach 24 h bezogen auf das Trockengewicht der Matrix absorbiert.

Bei elastomeren thermoplastischen Massen bezeichnet Kohäsion die Kräfte, die den Zusammenhalt der Masse bewirken. Diese Kohäsionskräfte sind zum einen für die Zähigkeit (Viskosität) und das Fließverhalten (Rheologie) der elastomeren Matrix bei der Verarbeitung und zum anderen für die Festigkeit der Matrix bei seiner Beanspruchung verantwortlich. Die Kohäsionskräfte in derartigem System werden durch Kennwerte wie E-Modul, Reißdehnung, Zug- und Reißfestigkeit/Reißkraft oder Shore-Härte beschrieben.

Zug- und Reißfestigkeitsprüfung:

Die Reißfestigkeit ist der Quotient aus der im Augenblick des Reißens gemessenen Kraft $F_R$ (Reißkraft) und dem Anfangsquerschnitt $A_0$ des Probekörpers, der an der dünnsten Stelle gemessen wird.

Die Zugfestigkeit ist der Quotient aus der gemessenen Höchstkraft (maximale Kraft) und dem Anfangsquerschnitt des Probekörpers, der an der dünnsten Stelle gemessen wird. Bei Elastomeren ist die Reißkraft im Allgemeinen auch die Höchstkraft.

Die Reißfestigkeit und die Reißdehnung der elastomeren Matrix wurde mit sterilen und unsterilen Prüfkörpern mit einem Anfangsquerschnitt $A_0$ von 100 mm$^2$ auf einer Zwick-Zugprüfmaschine gemessen. Bei der Ermittlung der Reißdehnung wird die Länge eines Probekörpers mit einer Ursprungslänge von 8 mm bei Erreichen der Reißkraft bestimmt.

Tabelle 1. Reißfestigkeit und Reißdehnung von sterilen und unsterilen Mustern

| Sterilisationsart | unsteril | | | Strahlensterilisation (Gamma) | | |
|---|---|---|---|---|---|---|
| Dosis | | | | 25-30kGy | | |
| Merkmal Muster | $F_R$max [N] | Reißfestigkeit [cN/mm$^2$] | Reißdehnung [mm] | $F_R$max [N] | Reißfestigkeit [cN/mm$^2$] | Reißdehnung [mm] |
| Beispiel 1 | 2,97 | 2,97 | 614 | 1,69 | 1,69 | 544 |
| Beispiel 1 | 2,80 | 2,80 | 579 | 1,48 | 1,48 | 395 |
| Beispiel 1 | 2,80 | 2,80 | 581 | 1,55 | 1,55 | 490 |
| Mittelwert | **2,86** | **2,86** | **591** | **1,57** | **1,57** | **476** |
| Beispiel 2 | 5,14 | 5,14 | 669 | 2,72 | 2,72 | 486 |
| Beispiel 2 | 4,69 | 4,69 | 686 | 3,09 | 3,09 | 516 |
| Beispiel 2 | 4,11 | 4,11 | 621 | 3,10 | 3,10 | 563 |
| Mittelwert | **4,65** | **4,65** | **659** | **2,97** | **2,97** | **522** |

Der Austausch an bereits geringen Mengen (e.g. 0,6%) des Elastomers mit hoher Molekularmasse (wie im Beispiel 1) durch ein ultrahochmolekulares Elastomer (wie im Beispiel 2) kompensiert vollständig den Abfall der Reißfestigkeit und der Kohäsion; erhöht damit die Beständigkeit der polymeren Elastomermatrix gegenüber Strahlensterilisation. (Musterbeispiel 1, unsteril 2,86 cN/mm$^2$; Musterbeispiel 2, steril 2,97 cN/mm$^2$). Auch der negative Einfluß der Strahlensterilisation auf die Reißdehnung wird zum Teil kompensiert.

Absorption von Salzlösung (NaCl 0,8 %) bei Wundauflagen nach 2h, 4h und 24h Kontaktzeit:

Eine 10 cm$^2$ große Probe wird mit einer Analysenwaage gewogen (w1). Eine Kristallisierschale wird so mit Salzlösung befüllt, daß die Probe komplett mit der Prüflösung bedeckt ist und sich nur wenige, kleine Luftblasen an ihr befinden. Zu bestimmten Zeitpunkten wird die Probe aus der Lösung entnommen und vorsichtig mit einem weichen Papiertuch abgetupft, bis alle Wassertropfen entfernt sind. Die trockene Probe wird mit der Analysenwaage gewogen (w2).

Die Berechnung der Absorption in %, bezogen auf Polymermatrixgewicht (Pg) erfolgt nach der folgenden Formel:

Hierfür muß das Trägergewicht (43 g/m$^2$) vom Probengewicht abgezogen werden.

$$\underline{\text{Absorption (Pg)} = (w2 - w1) / (w1 - 0{,}043) \times 100}$$

Tabelle 2. Absorption der Salin-Lösung nach 2, 4 und 24h

| Mittelwerte | Absorption (Pg) in % nach | | |
|---|---|---|---|
| | 2h | 4h | 24h |
| Beispiel 1 | 0,2 | 0,7 | 5,5 |
| Beispiel 2 | 0,9 | 2,7 | 9,7 |

Die Absorptionsdaten aus der Tabelle 2 demonstrieren eindeutig, daß bei einer besonders bevorzugten Ausführungsform der Erfindung die elastomere Wundkontaktschicht keine nennenswerte Menge an Salin-Lösung aufnimmt und somit nicht absorbierend ist. Die geringen Mengen an aufgenommenem Wasser werden zur Ausbildung einer nicht mit der Wunde verklebenden fett- und hydrokolloidgelhaltigen Grenzschicht genutzt.

Erfindungsgemäße Wundauflagen können wie folgt erhalten werden:

Beispiel 1:

| | Menge [g] | Gewichtsteile [%] |
|---|---|---|
| Paraffinöl | 1110 | 72,2 |
| Copolymer | 40 | 2,6 |
| Antioxidans | 1,5 | 0,1 |
| Vaseline | 154 | 10,0 |
| CMC | 231 | 15,0 |

Die Masse wird in einem Labordissolver hergestellt. 1110 g des Paraffinöls werden vorgelegt und mit 40 g eines Elastomer-Copolymers SEEPS (Septon 4055, Fa. Kuraray) und 1,5 g eines Antioxidans (Irganox 1010) versetzt und bei ca. 135°C bis zur Erhaltung einer homogenen klaren elastomeren Masse gerührt. Nach der Einarbeitung von 154 g Vaseline (Vara AB, Fa. Sasol) werden 231 g der Natrium-Carboxymethylcellulose (CMC, Blanose 7H4XF, Fa. Aqualon) zugesetzt. Die so erhaltene elastomere Masse wird noch etwa 30 min. gerührt, bis eine homogene Masse erhalten wird.

Die Masse kann ebenfalls in einem Kneter oder ähnlichen für die Verarbeitung von Hotmelt-Massen allg. bekannten Anlagen/Geräten hergestellt werden.

Die Masse kann in einem Tauchbad-Verfahren bei ca. 140 -145°C auf das Gewebe (Gittertüll) aufgebracht werden, so daß das textile Gebilde vollständig umhüllt ist, die Zwischenräume/Poren aber weitgehend offen bleiben, um den Durchfluß des Exsudats zu gewährleisten.

Beispiel 2:

Analog Bsp. 1, allerdings wurde ein Polymergemisch aus 2 % Septon 4055 und 0,6 % Septon 4077 eingesetzt.

Beispiel 3:

Analog Bsp. 1, allerdings wurden 15 % eines nichtionischen Cellulose-Derivats (HPMC, Bonucel D15000) eingesetzt.

Im Rahmen der Erfindung kann es von besonderer Bedeutung sein, daß die elastomere Wundkontaktschicht nur im Bereich von 5 % bis 30 % der Trockenmasse absorbiert. Dadurch wird die Gelbildung und Quellung (durch hydrokolloide Lösungen) eingeschränkt, so daß der Exsudattransport durch die Öffnungen des Verbands in den Sekundärverband nicht behindert wird.

Im Rahmen der Erfindung kann dem Gesichtspunkt besondere Bedeutung zukommen, daß der Träger offenmaschig ist, so daß nach der Beschichtung/Umhüllung der den Träger bildenden Fasern mit der elastomeren Masse noch ein Exsudatdurchfluß gewährleistet ist.

Die Erfindung ist nicht auf die vorstehend erläuterten Ausführungsbeispiele beschränkt. Vielmehr ist auch an den Einsatz von Trägermaterialien in Form von offenmaschigen Gewirken und Geweben und/oder Polyurethan-Schäumen wie etwa Vivo MCF 03 (Fa. AMS) gedacht. Dabei kann die erfindungsgemäße Wundauflage auch einen in der elastomeren Matrix gelösten/dispergierten/verteilten Wirkstoff auf Metallbasis, wie Silber, Kupfer, Selen, und/oder auf Basis von Metallverbindungen, insbesondere Metallsalze ($Ag_2O$, AgCl, ZnO, MgO), aufweisen.

Bei einer besonders bevorzugten Ausführungsform der Erfindung weist der Wirkstoff ein oligomeres/polymeres Biguanid, insbesondere PHMB (e.g. Cosmocil PQ, Arch) und/oder oligomeres/polymeres Guanid und/oder analoge Breitband-Antiseptika, wie Octenidin, auf.

**Patentansprüche**

1.  Sterile Wundauflage mit einem Träger und einer nicht absorbierenden elastomeren Wundkontaktschicht, **dadurch gekennzeichnet, daß** die elastomere Matrix durch ein synthetisches Dreiblock-Elastomer in Form eines Copolymers aus Polystyrolblock und Polyolefinblock (SEPS, SEBS, SEEPS, etc.) aus einer Mischung von einem hochmolekularen Polymer mit einem Molekulargewicht von 300.000 Dalton oder weniger und einem ultrahochmolekularen Polymer mit einem Molekulargewicht von mehr als 300.000 Dalton gebildet wird, wobei der Gesamtpolymeranteil unter 3,2 Gew.-% beträgt und durch ein unpolares Öl und/oder Vaseline plastifiziert ist und das Molekulargewicht des Dreiblock-Elastomers 150.000 Dalton oder mehr, aber weniger als 600.000 Dalton, beträgt, wobei der Anteil des ultrahochmolekularen Polymers am Gesamtpolymeranteil weniger als 50 Gew.-% beträgt und der Anteil des ultrahochmolekularen Polymers am Gesamtpolymeranteil mehr als 5 Gew.-% beträgt.

2.  Wundauflage nach Anspruch 1, **dadurch gekennzeichnet, daß** die elastomere Matrix mindestens ein synthetisches Dreiblock-Elastomer mit hohem Molekulargewicht und einer Brookfield-Viskosität von mindestens 5000 mPas. (für eine 10%ige Lösung in Toluol bei 30°C) enthält.

3.  Wundauflage nach einem der vorhergehenden Ansprüche, **dadurch gekennzeichnet, daß** die elastomere Matrix ein ionisches oder nichtionisches Hydrokolloid beinhaltet, das homogen in der Matrix dispergiert ist.

4.  Wundauflage nach einem der vorhergehenden Ansprüche, **dadurch gekennzeichnet, daß** der Träger ein vorzugsweise offenmaschiges Gewirk, Gewebe und/oder Vlies und/oder einen Polyurethan-Schaum aufweist.

5.  Wundauflage nach einem der vorhergehenden Ansprüche, **dadurch gekennzeichnet, daß** er unter anderem in seiner elastomeren Matrix einen Wirkstoff in einer therapeutisch wirksamen Menge umfaßt.

6.  Wundauflage nach einem der vorhergehenden Ansprüche, **dadurch gekennzeichnet, daß** der Wirkstoff homogen in der elastomeren Matrix gelöst/dispergiert/verteilt ist.

7.  Wundauflage nach einem der vorhergehenden Ansprüche, **dadurch gekennzeichnet, daß** die elastomere Matrix bereichsweise keinen Wirkstoff enthält.

8.  Wundauflage nach einem der vorhergehenden Ansprüche, **dadurch gekennzeichnet, daß** der Wirkstoff Metalle und/oder Metallverbindungen, insbesondere Metallsalze, umfaßt.

9.  Wundauflage nach einem der vorhergehenden Ansprüche, dadurch gekennzeichnit, daß der Wirkstoff ein oligomeres/polymeres Biguanid, insbesondere PHMB und/oder oligomeres/polymeres Guanid, und/oder analoge Breitband-Antiseptika, wie Octenidin, aufweist.

10. Wundauflage nach einem der vorhergehenden Ansprüche, **dadurch gekennzeichnet, daß** die elastomere Matrix einen Tackifier enthält, um die Klebkraft zu erhöhen.

**11.** Material zum Herstellen einer Wundkontaktschicht einer Wundauflage nach einem der vorhergehenden Ansprüche.

**Claims**

**1.** A sterile wound dressing comprising a backing and a non-absorbent elastomeric wound contact layer, **characterised in that** the elastomer matrix is formed by a synthetic three-block elastomer in the form of a copolymer comprising polystyrene block and polyolefin block (SEPS, SEBS, SEEPS, etc) from a mixture of a high molecular polymer with a molecular weight of 300,000 dalton or less and an ultra-high molecular polymer with a molecular weight of more than 300,000 dalton, the total polymer percentage being less than 3.2 % by weight and being plasticised by an apolar oil and/or petroleum jelly, and the molecular weight of the three-block elastomer being 150,000 dalton or more, but less than 600,000 dalton, the percentage of ultra-high molecular polymer in the total polymer percentage being less than 50 % by weight, and the fraction of ultra-high molecular polymer in the total polymer percentage being more than 5 % by weight.

**2.** The wound dressing according to Claim 1, **characterised in that** the elastomer matrix contains at least one synthetic three-block elastomer with a high molecular weight and a Brookfield viscosity of at least 5000 mPas (for a 10% solution in toluene at 30°C).

**3.** The wound dressing according to any of the preceding claims, **characterised in that** the elastomer matrix contains an ionic or non-ionic hydrocolloid that is dispersed homogeneously in the matrix.

**4.** The wound dressing according to any of the preceding claims, **characterised in that** the backing has a preferably open mesh knit, woven fabric and/or non-woven fabric and/or a polyurethane foam.

**5.** The wound dressing according to any of the preceding claims, **characterised in that** it comprises among other things in its elastomer matrix an active ingredient in a therapeutically effective amount.

**6.** The wound dressing according to any of the preceding claims, **characterised in that** the active ingredient is dissolved/dispersed/distributed homogeneously in the elastomer matrix.

**7.** The wound dressing according to any of the preceding claims, **characterised in that** the elastomer matrix does not contain any active ingredient in some areas.

**8.** The wound dressing according to any of the preceding claims, **characterised in that** the active ingredient comprises metals and/or metal compounds, in particular metal salts.

**9.** The wound dressing according to any of the preceding claims, **characterised in that** the active ingredient comprises an oligomeric/polymeric biguanide, in particular PHMB and/or oligomeric/polymeric guanide and/or similar broad spectrum antiseptics, such as octenidine.

**10.** The wound dressing according to any of the preceding claims, **characterised in that** the elastomer matrix contains a tackifier in order to increase the adhesive strength.

**11.** A material for producing a wound contact layer of a wound dressing according to any of the preceding claims.

**Revendications**

**1.** Pansement stérile comportant un support et une couche élastomère non absorbante destinée au contact avec la plaie, **caractérisé en ce que** la matrice élastomère se compose d'un élastomère tribloc synthétique sous la forme d'un copolymère composé d'un bloc polystyrène et d'un bloc polyoléfine (SEPS, SEBS, SEEPS, etc.), à partir d'un mélange d'un polymère de poids moléculaire élevé de l'ordre de 300 000 daltons ou moins et d'un polymère de poids moléculaire ultra-élevé de l'ordre de plus de 300 000 daltons, la teneur totale en polymères étant inférieure à 3,2 % en poids, et **en ce que** la matrice élastomère est plastifiée par une huile non polaire et/ou de la vaseline, et le poids moléculaire de l'élastomère tribloc est de 150 000 daltons ou plus, mais de moins de 600 000 daltons, la teneur en polymère de poids moléculaire ultra-élevé rapportée à la teneur totale en polymères étant de plus de 5 % en poids.

**2.** Pansement selon la revendication 1, **caractérisé en ce que** la matrice élastomère contient au moins un élastomère tribloc synthétique de poids moléculaire élevé et de viscosité Brookfield d'au moins 5000 mPa.s (pour une solution à 10 % dans le toluène à 30 °C).

**3.** Pansement selon l'une des revendications précédentes, **caractérisé en ce que** la matrice élastomère contient un hydrocolloïde ionique ou non ionique qui est dispersé de façon homogène dans la matrice.

**4.** Pansement selon l'une des revendications précédentes, **caractérisé en ce que** le support présente un voile et/ou un tricot et/ou un tissu à mailles de préférence ouvertes, et/ou une mousse polyuréthane.

**5.** Pansement selon l'une des revendications précédentes, **caractérisé en ce qu'**il comprend entre autres, dans sa matrice élastomère, un principe actif présent dans une quantité thérapeutiquement efficace.

**6.** Pansement selon l'une des revendications précédentes, **caractérisé en ce que** le principe actif est dissous/dispersé/réparti de manière homogène dans la matrice élastomère.

**7.** Pansement selon l'une des revendications précédentes, **caractérisé en ce que** la matrice élastomère ne contient aucun principe actif par endroits.

**8.** Pansement selon l'une des revendications précédentes, **caractérisé en ce que** le principe actif comprend des métaux et/ou des composés métalliques, notamment des sels métalliques.

**9.** Pansement selon l'une des revendications précédentes, **caractérisé en ce que** le principe actif présente du biguanide oligomère/polymère, notamment du PHMB et/ou du guanide oligomère/polymère, et/ou des antiseptiques à large spectre analogues tels que l'octénidine.

**10.** Pansement selon l'une des revendications précédentes, **caractérisé en ce que** la matrice élastomère contient un agent poisseux visant à accroître le pouvoir collant.

**11.** Matériau de production d'une couche de contact avec la plaie d'un pansement selon l'une des revendications précédentes.

**IN DER BESCHREIBUNG AUFGEFÜHRTE DOKUMENTE**

**In der Beschreibung aufgeführte Patentdokumente**

- EP 1691851 B1
- EP 1143895 B1
- EP 1143895 A
- EP 127229 B1
- EP 0521761 A
- FR 2783412 A1